# FASCICULE DE BREVET EUROPEEN

(11) **EP 3 260 086 B1**
(45) Date de publication et mention de la délivrance du brevet: **12.12.2018**
(21) Numéro de dépôt: 17176346.9
(22) Date de dépôt: 16.06.2017
(51) Int. Cl.: A61F 2/38

(54) **ENSEMBLE PROTHÉTIQUE POUR L'ARTICULATION DU GENOU**
PROTHESENEINHEIT FÜR KNIEGELENK
PROSTHETIC ASSEMBLY FOR THE KNEE JOINT

(30) Priorité: 22.06.2016 FR 1655792
(43) Date de publication de la demande: 27.12.2017
(73) Titulaire: Aston Medical, 42000 Saint-Etienne (FR)
(72) Inventeur: BURDIN, Gilles, 14740 ROSEL (FR); LE MEVEL, Philippe, 56800 PLOERMEL (FR); THIERRY, Jean-François, 13360 ROQUEVAIRE (FR); BUSCAYRET, Florent, 34090 MONTPELLIER (FR); LABATTUT, Ludovic, 21700 QUINCEY (FR); COLOMBIER, Michel, 34000 MONTPELLIER (FR); RASCLE, Christophe, 42480 LA FOUILLOUSE (FR)
(74) Mandataire: Cabinet Laurent & Charras

(56) Documents cités:
- EP-A1- 0 639 358
- FR-A1- 3 006 167
- US-A1- 2011 270 403
- US-A1- 2014 277 535

## Description

L'invention se rattache au secteur technique des implants orthopédiques et concerne, plus particulièrement, un ensemble prothétique pour l'articulation du genou. L'état de la technique le plus proche est le document FR 3006167 A1, qui définie le préambule de la revendication 1.

L'invention trouve une application avantageuse dans le cas d'une prothèse tricompartimentale du genou.

D'une manière parfaitement connue pour un homme du métier, ce type de prothèse comprend un implant fémoral et un implant tibial, présentant tous types d'agencements, pour être fixés sur la partie articulaire anatomique de l'os correspondant, après y avoir effectué des coupes fémorales et des coupes tibiales nécessaires.

L'implant fémoral comprend une trochlée prolongée, de part et d'autre d'une échancrure, par deux patins condyliens en appui, avec capacité de flexion et de rotation, avec des empreintes congruentes complémentaires, que présente un plateau tibial, généralement réalisé en polyéthylène.
Le plateau tibial est monté avec capacité de mobilité sur une embase tibiale.

A partir de cette conception de base de prothèse d'articulation du genou, il est connu différentes formes de réalisation, notamment au niveau de l'articulation de l'implant fémoral et de l'implant tibial, en fonction du cas pathologique à traiter.

Par exemple, dans le cas de reprise d'une prothèse du genou, le chirurgien doit disposer, en fonction de l'état des ligaments latéraux, soit d'une prothèse hautement postéro-stabilisée étant donné que les ligaments croisés n'existent plus, soit d'une prothèse à pivot ou charnière, afin de stabiliser l'articulation du genou lorsqu'il est nécessaire de remplacer tout le système ligamentaire.

Par conséquent il est nécessaire de disposer de deux types de prothèses, et de deux ancillaires pour la pose de la prothèse considérée.
Il en résulte d'importantes quantités d'implants et d'instruments, nécessaires à l'adaptation de la prothèse au cas pathologique à traiter.
Bien évidemment, il en résulte également des coûts importants.

L'invention s'est fixée pour but de remédier à ces inconvénients, de manière simple, sûre, efficace et rationnelle.

Le problème que se propose de résoudre l'invention est de diminuer de manière significative le nombre de pièces, en ayant pour objectif de transformer à volonté la prothèse, pour obtenir en fonction du cas pathologique à traiter, soit une prothèse hautement postéro-stabilisée, soit une prothèse dite à pivot ou à charnière.

Pour résoudre un tel problème, il a été conçu un ensemble prothétique pour l'articulation du genou comprenant un implant fémoral et un implant tibial :
- l'implant fémoral présente une trochlée prolongée de part et d'autre d'une échancrure, par deux patins condyliens, ladite échancrure étant prolongée par une cage reliant les bords internes des patins condyliens et la trochlée,
- l'implant tibial présente une embase tibiale, avec en débordement, un plot de rotation pour le montage avec capacité de rotation d'un plateau tibial,
les patins condyliens coopèrent avec capacité de flexion et de rotation avec des empreintes congruentes du plateau tibial.

Selon l'invention, compte tenu du problème à résoudre, la cage présente des agencements pour recevoir à volonté, en fonction des cas pathologiques à traiter :
- soit un ensemble pivot apte à être engagé au travers d'une ouverture que présente un premier plateau tibial, sur le plot de rotation de l'embase tibiale, pour obtenir une prothèse à pivot,
- soit un ensemble faisant office de came, apte à coopérer avec une surface congruente d'un bossage, formé en débordement d'un deuxième plateau tibial de manière à être centré entre les deux patins condyliens, pour obtenir une prothèse postéro-stabilisée,
l'implant fémoral et l'embase tibiale étant communs aux deux types de prothèses.

Il résulte donc de ces caractéristiques, que pour obtenir une prothèse hautement postéro-stabilisée, ou une prothèse à pivot, l'implant fémoral et l'embase tibiale sont les mêmes, seul le plateau tibial et ses moyens de liaison avec l'implant fémoral, sont différents.

Pour résoudre le problème posé d'assurer l'accouplement de l'ensemble pivot à l'implant fémoral, l'ensemble pivot comprend un insert présentant des moyens d'accouplement temporaires aux agencements de la cage de l'implant fémoral, ledit insert formant une chape pour le montage à libre articulation d'un pivot au moyen d'un axe.

L'axe d'articulation du pivot est engagé transversalement au travers de trous formés dans l'épaisseur de la cage, les ailes de la chape de l'insert, et une zone du pivot déportée angulairement par rapport à une zone apte à coopérer avec le plot de rotation de l'embase tibiale.

Selon une autre caractéristique, la zone du pivot apte à coopérer avec le plot de rotation de l'embase tibiale est constituée par une portée cylindrique creuse recevant un fourreau destiné à être à libre rotation sur ledit plot.

Pour configurer l'ensemble prothétique en prothèse postéro stabilisée, l'ensemble faisant office de came, présente des moyens d'accouplement temporaire avec les agencements de la cage de l'implant fémoral, et reçoit un axe d'articulation engagé transversalement au travers de trous, formés dans l'épaisseur de la cage de l'implant fémoral, et au travers d'un alésage débouchant que présente la came.

La came et le pivot sont accouplés à l'élément fémoral au moyen d'un même axe.

Aussi bien dans le cas d'une prothèse à pivot, que dans le cas d'une prothèse hautement postéro-stabilisée, l'axe présente un épaulement circulaire coopérant avec un lamage, que présente l'un des trous de la cage de l'implant fémoral.

Pour résoudre le problème posé d'assurer l'accouplement de l'ensemble pivot ou de l'ensemble came à l'implant fémoral, les moyens d'accouplement temporaires de l'insert recevant le pivot et de la came, sont constitués par des nervures et bossages coopérant avec des rainures que présentent les faces internes en regard des parois verticales de la cage de l'implant fémoral.

Suivant une autre caractéristique, l'implant fémoral et l'embase tibiale présentent des agencements pour recevoir une tige d'ancrage apte à être engagée dans le canal médullaire du fémur et du tibia.

L'invention est exposée ci-après plus en détail à l'aide des figures des dessins annexés dans lesquels :
- la figure 1 est une vue, avant assemblage, des principaux éléments de l'ensemble prothétique pour la réalisation d'une prothèse hautement postéro stabilisée,
- la figure 2 est une vue, avant assemblage, des principaux éléments, dans le cas d'un exemple de réalisation d'une prothèse du genou hautement postéro stabilisée,
- la figure 3 est une vue en perspective correspondant à la figure 2, après assemblage des différents éléments constitutifs de la prothèse,
- la figure 4 est une vue en perspective, avant assemblage, des principaux éléments pour la réalisation d'une prothèse du genou à pivot,

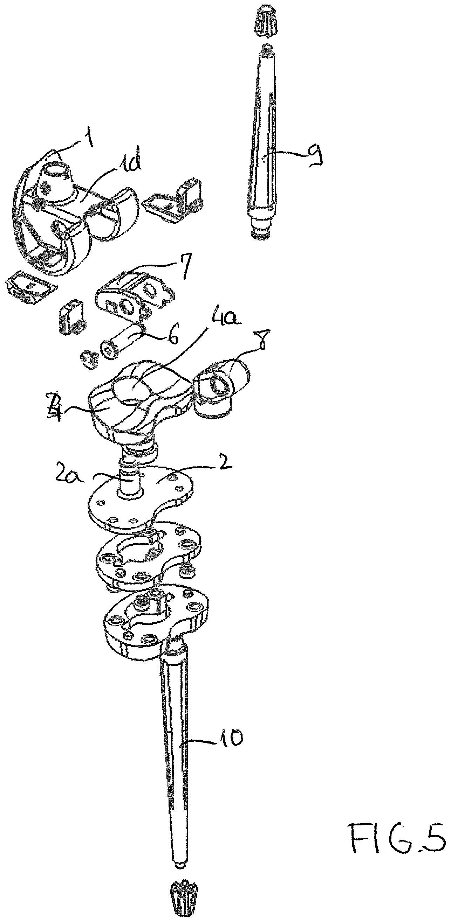

- la figure 5 une vue en perspective, avant assemblage, des principaux éléments dans le cas d'un exemple de réalisation d'une prothèse totale du genou à pivot,
- la figure 6 est une vue en perspective correspondant à la figure 5,
- la figure 7 est une vue en coupe longitudinale correspondant à la forme de réalisation de la prothèse, illustrée figure 3,
- la figure 8 est une vue en coupe, selon la ligne K-K de la figure 7,

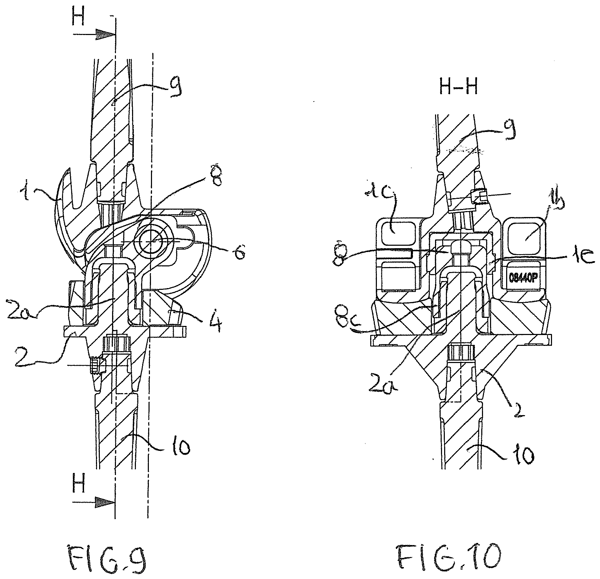

- la figure 9 est une vue en coupe longitudinale correspondant à la forme de réalisation de la prothèse illustrée figure 4,
- la figure 10 est une vue en coupe selon la ligne H-H de la figure 9,
- la figure 11 est une vue en perspective de l'implant fémoral avant mise en place, soit de la came, soit de l'insert, recevant le pivot.

De manière parfaitement connue pour un homme du métier, l'ensemble prothétique pour l'articulation du genou comprend un implant fémoral désigné de son ensemble par (F), et un implant tibial désigné dans son ensemble par (T). L'implant fémoral (F) présente une trochlée (1) prolongée de part et d'autre d'une échancrure (1a), par deux patins condyliens (1b) et (1c). Par ailleurs l'échancrure (1a) est prolongée par une cage intercondylienne (1d) reliant les bords internes des patins condyliens (1b) et (1c) et la trochlée (1).

L'implant tibial (T) présente une embase tibiale (2) avec, en débordement, un plot de rotation (2a) pour le montage, avec capacité de rotation, d'un plateau tibial (3) ou (4), comme il sera indiqué dans la suite de la description.

Selon invention, le but recherché est de permettre au chirurgien, à partir d'un même implant fémoral (F) et de la même embase tibiale (2), de pouvoir réaliser, en fonction du cas pathologique à traiter, notamment de l'état ligamentaire, soit une prothèse hautement postéro-stabilisée, soit une prothèse à pivot ou à charnière.

Plus particulièrement, pour atteindre cet objectif, la cage (1d) de l'implant fémoral (F), présente des agencements pour recevoir, à volonté, soit un ensemble faisant office de came pour obtenir une prothèse hautement postéro-stabilisée, soit un ensemble pivot, pour obtenir une prothèse à charnière où à pivot.

L'ensemble faisant office de came coopère avec un plateau tibial (3) tandis que l'ensemble pivot coopère avec un plateau tibial (4). Il apparaît donc, qu'en fonction du type de prothèse à obtenir, soit postéro-stabilisée, soit à pivot, seul le plateau tibial est différent, l'implant fémoral (F) et l'embase tibiale (2) demeurant identiques dans les deux cas.

L'ensemble faisant office de came est apte à coopérer avec une surface congruente (3a) d'un bossage (3b) formé en débordement du côté du plateau tibial (2), de manière à être centré entre les patins condyliens (1b) et (1c).

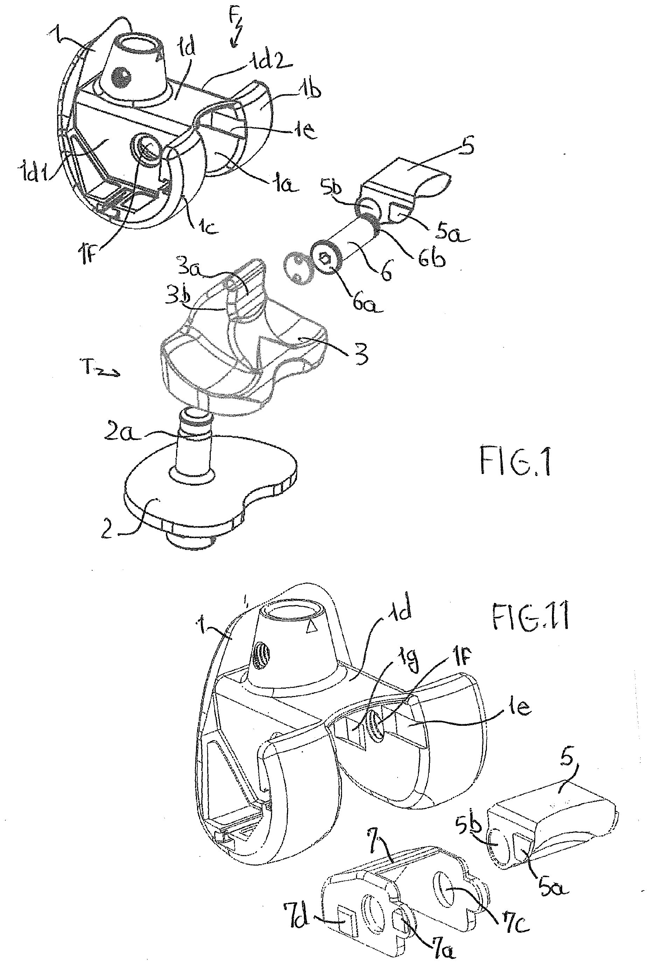

L'ensemble faisant office de came, est constitué par un élément indépendant (5) constituant la came en tant que telle. Cet élément (5) présente des moyens d'accouplement temporaire, avec des agencements de la cage (1d) de l'implant fémoral. Ces moyens d'accouplement temporaires sont par exemple constitués par des bossages (5a) formés en débordement des côtés de l'élément (5), et coopérant avec des rainures condyliennes postérieures complémentaires (le) que présentent les faces internes en regard des parois verticales (1d1) et (1d2) de la cage (1d) de l'implant fémoral (figure 11).

Le montage de la came (5) à l'intérieur de la cage (1d) s'effectue au moyen d'un axe (6) engagé transversalement au travers de trous ou orifices (1f) formés dans l'épaisseur des parois (1d1) et (1d2) de la cage (1d) et au travers d'un alésage débouchant (5b) que présente la came (5). Cette came (5) vient en contact avec la surface congruente (3a) du bossage (3d) du plateau tibial (3). Le plateau tibial (3) présente au niveau du bossage (3b), un chambrage interne (3c) apte à coopérer avec le plot (2a) de l'embase tibiale (2), permettant ainsi la rotation du plateau lors de la flexion.

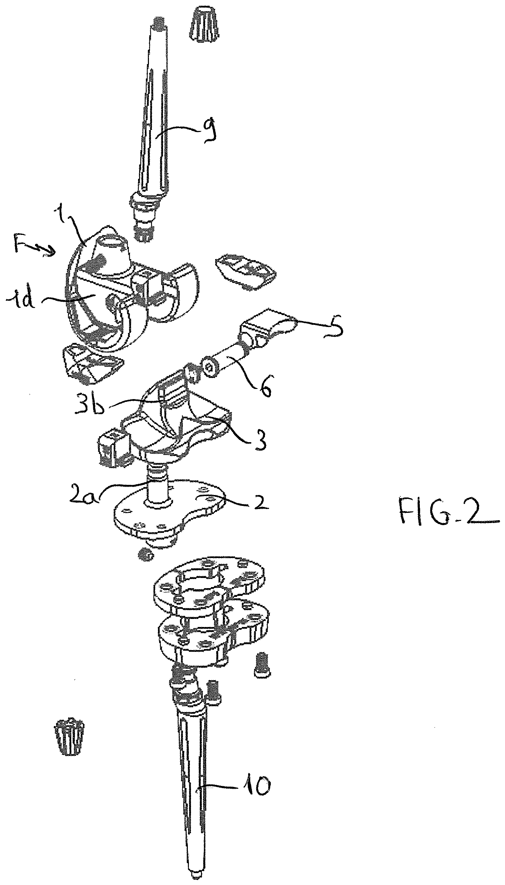

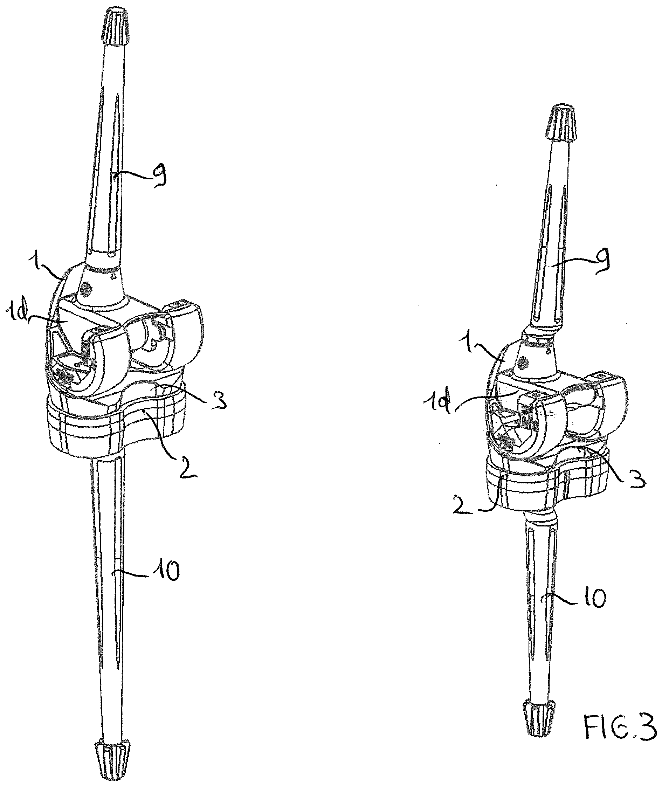

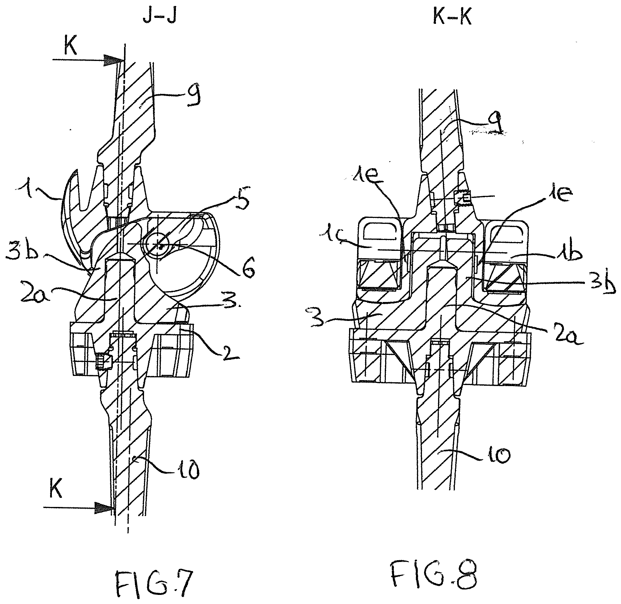

Ces différentes caractéristiques permettent donc d'obtenir une prothèse totale du genou, hautement postéro-stabilisée, lorsque les ligaments croisés n'existent plus. On renvoie aux figures 1 à 3 et 7, 8 des dessins.

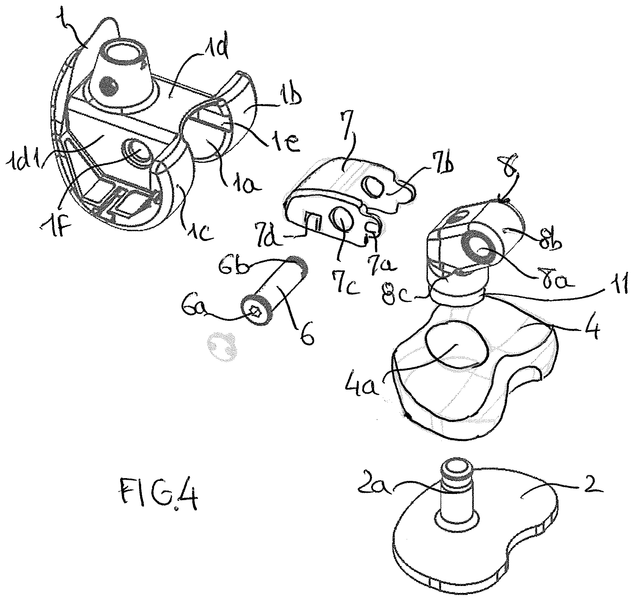

Pour réaliser une prothèse tricompartimentale du genou, dite à charnière ou à pivot, l'ensemble pivot est engagé au travers d'une ouverture (4a) que présente le plateau tibial (4) afin de coopérer avec le plot de rotation (2a) de l'embase tibiale (2). Comme le montre notamment la figure 4, l'ensemble pivot comprend un insert (7) en polyéthylène présentant des agencements d'accouplement temporaire avec les agencements de la cage (1d) de l'implant fémoral, de façon similaire à la came (5). Les agencements de l'insert (7) sont constitués par deux bossages (7a) et (7d) formés en débordement des côtés de l'insert (7) et coopérant avec les rainures condyliennes postérieures complémentaires (le) et des rainures condyliennes antérieures (1g) que présentent les faces internes en regard des parois verticales de la cage (1d) de l'élément fémoral (figure 11).

L'insert (7) présente d'un côté, une chape (7b) pour le montage à libre articulation d'un pivot (8) au moyen de l'axe (6). De la même façon que pour la came (5), l'axe (6) est engagé transversalement au travers des orifices (1f) formés dans l'épaisseur de la cage (1d) et au travers d'orifices (7c) formés dans les ailes de la chape (7b), et au travers d'un alésage débouchant (8d) formé transversalement dans une zone (8b). Cette zone (8b) est déportée postérieurement par rapport à une zone (8c) apte à être engagée à libre rotation, par l'intermédiaire d'un fourreau en polyéthylène (11), avec le plot (2a) de l'embase tibiale (2), après avoir été engagée au travers de l'ouverture (4a) du plateau tibial (4), sur le plot de rotation (2b) de l'embase tibiale (2).La zone (8c) est polie de façon à avoir une libre rotation autour de l'ouverture (4a) du plateau tibial (4).

Ces différentes caractéristiques permettent donc d'obtenir une prothèse tricompartimentale du genou, du type à charnière avec montage rotatoire du plateau tibial (4) par rapport à l'embase tibiale (2) avec pour objectif, de remplacer tout le système ligamentaire.

À noter également que le plateau tibial (4) peut reculer en fonction de la flexion du genou, comme le font les ménisques d'un genou sain, grâce à l'ouverture oblongue (4a).

Dans une forme de réalisation, l'axe de liaison (6) présente une tête circulaire (6a) coopérant avec un lamage que présente l'un des trous (1f) de la cage (1d) de l'implant fémoral. L'extrémité (6b) de l'axe présente un filetage pour pouvoir visser l'ensemble en compression.

Bien évidemment, la fixation de l'implant fémoral et de l'implant tibial au niveau des coupes distale, fémorale et tibiale, peut être effectuée par tous moyens connus et appropriés. Par exemple, comme illustré sur les dessins, l'implant fémoral et l'embase tibiale présentent respectivement des agencements pour recevoir une tige d'ancrage (9) et (10) apte à être engagée respectivement dans le canal médullaire du fémur et dans le canal médullaire du tibia.

Les avantages ressortent bien de la description. En particulier, on souligne et on rappelle :
- la possibilité, en per-opératoire, à partir d'un même implant fémoral et d'une même embase tibiale, d'obtenir en fonction de l'état ligamentaire du genou, soit une prothèse hautement postéro stabilisée, soit une prothèse à pivot en changeant seulement les composants condyliens (ensemble pivot ou ensemble faisant office de came).

## Revendications

1. Ensemble prothétique pour l'articulation du genou comprenant un implant fémoral (F) et un implant tibial (T):
- l'implant fémoral présente une trochlée (1) prolongée de part et d'autre d'une échancrure (1a), par deux patins condyliens (1b) et (1c), ladite échancrure intercondylienne (1a) étant prolongée par une cage (1d) reliant les bords internes des patins condyliens et la trochlée,
- l'implant tibial présente une embase tibiale (2) avec, en débordement, un plot de rotation (2a) pour le montage avec capacité de rotation d'un plateau tibial (3) ou (4),
- les patins condyliens coopèrent avec capacité de flexion et de rotation avec des empreintes congruentes du plateau tibial (3) ou (4),
***caractérisé en ce que*** la cage (1d) présente des agencements pour recevoir à volonté, en fonction des cas pathologiques à traiter :
- soit un ensemble pivot (7)-(8) apte à être engagé au travers d'une ouverture (4a) que présente un premier plateau tibial (4), sur le plot de rotation (2a) de l'embase tibiale (2), pour obtenir une prothèse à pivot,
- soit un ensemble faisant office de came (5), apte à coopérer avec une surface congruente (3a) d'un bossage (3b) formé en débordement d'un deuxième plateau tibial (3), de manière à être monté entre les deux patins condyliens (1b) et (1c), pour obtenir une prothèse postéro-stabilisée,
l'implant fémoral (F) et l'embase tibiale (2) étant communs aux deux types de prothèses.

2. Ensemble selon la revendication 1, ***caractérisé* en ce que** l'ensemble pivot comprend un insert (7) présentant des moyens d'accouplement temporaires aux agencements de la cage de l'implant fémoral, ledit insert (7) formant une chape (7b) pour le montage à libre articulation d'un pivot (8) au moyen d'un axe (6).

3. Ensemble selon la revendication 2, ***caractérisé* en ce que** l'axe d'articulation (6) du pivot (8) est engagé transversalement au travers de trous formés dans l'épaisseur de la cage (1d), des ailes de la chape (7b) de l'insert (7), et d'une zone (8b) du pivot (8) déportée postérieurement par rapport à une zone (8c) apte à coopérer avec le plot de rotation (2a) de l'embase tibiale (2).

4. Ensemble selon la revendication 3, ***caractérisé* en ce que** la zone (8c) du pivot (8) apte à coopérer avec le plot de rotation (2a) de l'embase tibiale (2), est constituée par une portée cylindrique creuse recevant un fourreau en polyéthylène destiné à être monté à libre rotation sur ledit plot.

5. Ensemble selon la revendication 1, ***caractérisé* en ce que** l'ensemble faisant office de came (5), présente des moyens d'accouplement temporaire avec les agencements de la cage (1d) de l'implant fémoral, et reçoit un axe d'articulation (6) engagé transversalement au travers de trous, formés dans l'épaisseur de la cage de l'implant fémoral, et au travers d'un alésage débouchant (5b) que présente la came (5).

6. Ensemble selon l'une quelconque des revendications 1-5, ***caractérisé* en ce que** la came (5) et le pivot (8) sont accouplés à l'élément fémoral au moyen d'un même axe (6).

7. Ensemble selon la revendication 6, ***caractérisé* en ce que** l'axe présente un épaulement circulaire (6a) coopérant avec un lamage, que présente l'un des trous de la cage de l'implant fémoral, et à l'opposé un filetage (6b) pour immobiliser l'ensemble.

8. Ensemble selon l'une quelconque des revendications 1-7, ***caractérisé* en ce que** les moyens d'accouplement temporaires de l'insert (7) recevant le pivot (8), sont constitués par des bossages (7a) et (7d) coopérant avec des rainures condyliennes postérieures (le) et antérieures (1g) que présentent les faces internes en regard des parois verticales de la cage de l'implant fémoral.

9. Ensemble selon l'une quelconque des revendications 1-7, ***caractérisé* en ce que** les moyens d'accouplement temporaires de la came (5) sont constitués par des bossages (5a) coopérant avec des rainures condyliennes postérieures (le) que présentent les faces internes en regard des parois verticales de la cage de l'implant fémoral.

10. Ensemble selon l'une quelconque des revendications 1-9, ***caractérisé* en ce que** l'implant fémoral et l'embase tibiale présentent des agencements pour recevoir une tige d'ancrage (9) et (10) apte à être engagée dans le canal médullaire du fémur et du tibia.

## Patentansprüche

1. Prothesenanordnung für das Kniegelenk mit einem
Femurimplantat (F) und ein Tibialimplantat (T), wobei
• das Femurimplantat eine Trochlea (1) aufweist, die auf beiden Seiten einer Kerbe (1a) durch zwei Kondylschlitten (1b) und (1c) verlängert ist, wobei die interkondyläre Kerbe (1a) durch einen Käfig (1d) verlängert ist, der die Innenkanten der Kondylschlitten und der Trochlea verbindet,
• das Tibialimplantat eine Tibiabasis (2) mit, darüber hinausragend, einen Rotationszapfen (2a) zur drehfähigen Montage einer Tibiaplatte (3) oder (4),
• wobei die Kondylschlitten beuge- und drehfähig mit den kongruenten Abdrücken der Tibiaplatte (3) oder (4) zusammenwirken,
**dadurch gekennzeichnet, dass** der Käfig (1d) Vorrichtungen umfasst, die, abhängig von den zu behandelnden pathologischen Fällen, zur beliebigen Aufnahme von folgenden Elementen dienen:
• entweder eine Schwenkanordnung (7)-(8), die durch eine Öffnung (4a), die eine erste Tibiaplatte (4) aufweist, auf dem Rotationspolster (2a) der Tibiabasis (2) eingreifen kann, um eine Drehprothese zu erhalten,
oder eine als Nocken (5) wirkende Anordnung, die in der Lage ist, mit einer kongruenten Oberfläche (3a) einer Nabe (3b) zusammenzuwirken, die durch Überlaufen eines zweiten Tibiaplateaus (3) gebildet ist, um zwischen den beiden Kondylenpolstern (1b) und (1c) montiert zu werden, um eine posterior stabilisierte Prothese zu erhalten,
wobei das Femurimplantat (F) und die Tibiabasis (2) für beide Arten von Prothesen gemeinsam sind.

2. Anordnung nach Anspruch 1, **dadurch gekennzeichnet, dass** die Schwenkanordnung einen Einsatz (7) mit temporären Kopplungsmitteln an die Femurimplantatkäfiganordnungen umfasst, wobei der Einsatz (7) einen Gabelkopf (7b) zur frei klappbaren Befestigung eines Gelenks (8) mittels einer Achse (6) bildet.

3. Zusammen nach Anspruch 2, **dadurch gekennzeichnet, dass** die Scharnierachse (6) des Drehgelenks (8) quer durch Löcher, die in der Dicke des Käfigs (1d) ausgebildet sind, in den Flügeln des Gabelkopfes (7b) des Einsatzes (7) und einem Bereich (8b) des Drehgelenks (8), der später von einem Bereich (8c) versetzt ist, der mit dem Drehbolzen (2a) der Tibiabasis (2) zusammenwirken kann, in Eingriff steht.

4. Zusammen nach Anspruch 3, **dadurch gekennzeichnet, dass** der Bereich (8c) des Drehpunktes (8), der mit dem Rotationskissen (2a) der Tibiabasis (2) zusammenwirken kann, aus einem hohlzylindrischen Lager besteht, das eine Polyethylenhülse aufnimmt, die dazu bestimmt ist, frei drehbar auf dem Kissen montiert zu werden.

5. Anordnung nach Anspruch 1, **dadurch gekennzeichnet, dass** die als Nocken (5) wirkende Anordnung Mittel zur temporären Kopplung mit den Anordnungen des Käfigs (1d) des Oberschenkelimplantats aufweist und einen Scharnierstift (6) aufnimmt, der quer durch die in der Dicke des Käfigs des Oberschenkelimplantats ausgebildeten Löcher und durch ein Durchgangsloch (5b) des Nockens (5) greift.

6. Zusammen nach einem der Ansprüche 1-5, **dadurch gekennzeichnet, dass** der Nocken (5) und der Drehpunkt (8) über die gleiche Achse (6) mit dem Femurelement gekoppelt sind.

7. Zusammen nach Anspruch 6, **dadurch gekennzeichnet, dass** die Achse eine kreisförmige Schulter (6a), die mit einem Senker zusammenwirkt, der eines der Löcher des Oberschenkelimplantatkäfigs aufweist, und umgekehrt ein Gewinde (6b) zum Immobilisieren der Baugruppe aufweist.

8. Zusammen nach einem der Ansprüche 1-7, **dadurch gekennzeichnet, dass** die temporären Kopplungsmittel des Einsatzes (7), der den Stift (8) aufnimmt, aus Naben (7a) und (7d) bestehen, die mit hinteren (1e) und vorderen (1g) Kondylenrillen zusammenwirken, die die Innenflächen zu den vertikalen Wänden des Oberschenkelimplantatkäfigs darstellen.

9. Zusammen nach einem der Ansprüche 1-7, **dadurch gekennzeichnet, dass** die temporären Kopplungsmittel der Nocke (5) aus Naben (5a) bestehen, die mit hinteren Kondylenrillen (1e) zusammenwirken, die die Innenflächen zu den vertikalen Wänden des Oberschenkelimplantatkäfigs aufweisen.

10. Zusammen nach einem der Ansprüche 1-9, **dadurch gekennzeichnet, dass** das Oberschenkelimplantat und die Tibiabasis Vorkehrungen zur Aufnahme einer Ankerstange (9) und (10) aufweisen, die in den Markraum des Oberschenkelknochens und der Tibiae eingreifen können.

## Claims

1. Prosthetic assembly for the articulation of the knee, incorporating a femoral implant (F) and a tibial implant (T):
• the femoral implant has a trochlea (1) with two condylar pads (1b) and (1c) extending on either side of a cleft (1a), with the said intercondylar cleft (1a) having a cage (1d) extending from it, connecting the interior edges of the condylar pads and the trochlea;
• the tibial implant has a tibial base (2) with, a rotation stud (2a) overlapping, for the mounting of a tibial plate (3) or (4) with a rotation capability;
• the condylar pads interoperate, with a flexing and rotation capability, with the congruent imprints of the tibial plate (3) or (4);
***characterized in that*** the cage (1d) has fittings to accommodate, on demand, according to the pathological cases to be treated:
• either a pivot assembly (7)-(8) that can be engaged through an opening (4a) in a first tibial plate (4) onto the rotation stud (2a) of the tibial base (2), in order to procure a prosthesis with pivot;
• or an assembly acting as a cam (5) that is able to interoperate with a congruent surface (3a) of a lobe (3b) existing in an overlapping arrangement on a second tibial plate (3), so as to be mounted between the two condylar pads (1b) and (1c), in view of procuring a postero-stabilized prosthesis;
with the femoral implant (F) and the tibial base (2) being common to the two types of prosthesis.

2. Assembly in accordance with claim 1, ***characterized in that*** the pivot assembly incorporates an insert (7) being endowed with means of temporary coupling to the fittings of the cage of the femoral implant, with the said insert (7) forming a yoke (7b) for the mounting of a pivot (8) in free articulation, by means of a pin (6).

3. Assembly in accordance with claim 2, ***characterized in that*** the articulation pin (6) of the pivot (8) is engaged transversally through holes existing within the thickness of the cage (1d), in the fins of the yoke (7b) of the insert (7), and an area (8b) of the pivot (8) set rearwards in relation to an area (8c) able to interoperate with the rotation stud (2a) of the tibial base (2).

4. Assembly in accordance with claim 3, ***characterized in that*** the area (8c) of the pivot (8) able to interoperate with the rotation stud (2a) of the tibial base (2) is composed of a hollow cylindrical span accommodating a sleeve in polyethylene designed to be mounted on the said stud in free rotation.

5. Assembly in accordance with claim 1, ***characterized in that*** the assembly acting as a cam (5) has means of temporary coupling with the fittings of the cage (1d) of the femoral implant, and accommodates an articulation pin (6) engaged transversally through holes existing within the thickness of the cage of the femoral implant, and through an opening bore (5b) in the cam (5).

6. Assembly in accordance with any one of claims 1 to 5, ***characterized in that*** the cam (5) and the pivot (8) are coupled to the femoral part by means of one single pin (6).

7. Assembly in accordance with claim 6, ***characterized in that*** the pin has a circular shoulder (6a) interoperates with a counterbore in one of the holes of the cage of the femoral implant, and opposite a threading (6b) to immobilize the assembly.

8. Assembly in accordance with any one of claims 1 to 7, ***characterized in that*** the means of temporary coupling of the insert (7) accommodating the pivot (8) consist of bosses (7a) and (7d) interoperating with the posterior (1e) and anterior (1g) condylar channels existing on the interior faces opposite the vertical walls of the cage of the femoral implant.

9. Assembly in accordance with any one of claims 1 to 7, ***characterized in that*** the means of temporary coupling of the cam (5) consist of bosses (5a) interoperating with the posterior condylar channels (1e) existing in the interior faces opposite the vertical walls of the cage of the femoral implant.

10. Assembly in accordance with any one of claims 1 to 9, ***characterized in that*** the femoral implant and the tibial base are endowed with fittings to accommodate an anchoring rod (9) and (10) that can be engaged in the medullary cavity of the femur and tibia.
